# EUROPEAN PATENT APPLICATION

(11) **EP 2 206 429 A1**
(43) Date of publication of application: **14.07.2010**
(21) Application number: 08835180.4
(22) Date of filing: 29.09.2008
(51) Int. Cl.: A01N 25/08, A01N 25/04, A01N 43/40, A01N 55/02, A01N 55/08, A01N 59/16, A01N 59/20, A01P 3/00, A01P 17/00, A61K 8/49, A61Q 5/00, C09D 5/16, C09D 7/12, C09D 201/00

(54) **ANTIBIOTIC COMPOSITION AND PROCESS FOR PRODUCTION OF THE SAME**

(30) Priority: 01.10.2007 JP 2007280961
(71) Applicant: YHS Ltd., Sakai-shi, Osaka 5900114 (JP)
(72) Inventor: HIDAKA, Yasuhiro, Sakai-shi Osaka 590-0114 (JP)
(74) Representative: von Kreisler Selting Werner
(86) International application number: PCT/JP2008/002704
(87) International publication number: WO 2009/044523

(57) **Abstract**

A new technology is demanded to reduce the amount of zinc pyrithione added to hair care products as an anti-dandruff agent so as to reduce an irritation of the ocular mucosa, and to decrease an environmental influence in rivers, lakes and ponds which wastewater containing zinc pyrithione flows into. It is also demanded to accelerate the degradation of zinc pyrithione in sewage treatment facilities and the above environment.

Furthermore, zinc pyrithione formulated as an antifouling agent in ship-bottom paints is leached from a coating film at a rather higher rate and thus results in poor long-term antifouling effect, so that it is also demanded to lower the solubility thereof in seawater.

These problems could be solved at the same time by an antibiotic composition obtained by adsorbing zinc pyrithione or a zinc pyrithione-zinc oxide complex compound to a titanium oxide hydrate.

## Description

### TECHNCIAL FIELD

The present invention relates to an antibiotic composition comprising zinc pyrithione or a zinc pyrithione-zinc oxide complex compound and a titanium oxide hydrate, and a process for producing the same.

### BACKGROUND ART

Commonly, metal-adsorptive capacity and ion-exchanging capacity of a titanium oxide hydrate are well known. An attempt has been made to develop it as an agent extracting uranium in seawater, utilizing the characteristics (Non-Patent Document 1). An antimicrobial agent including silver ions adsorbed in a titanium oxide hydrate is also known (Patent Document 1). However, neither an antibiotic composition comprising a titanium oxide hydrate and an organo-metallic salt or an organo-metallic complex adsorbed to it, nor an antibiotic composition comprising a titanium oxide hydrate and zinc pyrithione adsorbed to it is known.
Patent Document 1:
   Japanese Patent Application Publication (Kokai) No. 6-298532
Non-Patent Document 1:
   Encyclopedia of Chemical Technology, 4th ed., Vol. 24, pp.235

### DISCLOSURE OF THE INVENTION

### Problems to be solved by the invention

Zinc pyrithione has hitherto been used as an anti-dandruff agent over 40 years. However, a new technology is demanded to reduce the amount of zinc pyrithione added to hair care products such as shampoo so as to reduce an irritation of the ocular mucosa and to decrease an environmental influence in river water which wastewater containing zinc pyrithione flows into.
Furthermore, zinc pyrithione formulated as an antifouling agent in ship-bottom paints is leached from a coating film at a rather higher rate, so that it is also demanded to lower the solubility thereof in seawater so as to_ produce the long-term antifouling effect.

### Means for Solving the Problems

In order to solve these problems, the inventor of the present invention has conceived that when zinc pyrithione is used in combination with other synergetic substances to be adsorbed to it, it might be possible to enhance the antibiotic activity, more specifically the anti-microbial activity against Malassezia furfur which cause dandruff on the scalp and to lower the solubility thereof in seawater, and thus enabling inhibition of breeding and growth of marine organisms such as barnacles, shellfishes and seaweeds which are adhered to and grow on such as coating film at ship-bottom and fish-farming nets.

The inventor of the present invention has intensively studied so as to solve these problems and found that these problems can be solved at the same time with a composition obtained by mixing zinc pyrithione or a zinc pyrithione-zinc oxide complex compound represented by the general formula (I) of the present invention with a titanium oxide hydrate, preferably adsorbing the zinc pyrithione or a zinc pyrithione-zinc oxide complex compound to the titanium oxide hydrate. Thus, the present invention has been completed.

The present invention provides:
(1) An antibiotic composition comprising:
   zinc pyrithione or a zinc pyrithione-zinc oxide complex compound represented by the general formula (I): 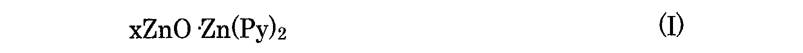 wherein Py represents 2-pyridylthio-N-oxide, and x represents 0 or a positive number, which satisfies a relation: 0 ≤ x ≤ 1, and a titanium oxide hydrate represented bv the general formula (II): 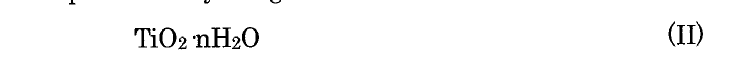 wherein n represents 1 or 2;
(2) The antibiotic composition according to (1), which is an anti-dandruff agent;
(3) The antibiotic composition according to (1), which is an underwater antifouling agent;
(4) The antibiotic composition according to (3), which further contains a binder, an inorganic copper compound, and/or an inorganic zinc compound, and/or copper pyrithione, and/or a triphenylborane compound;
(5) Aprocess for producing an antibiotic composition, which comprises stirring zinc pyrithione or a zinc pyrithione-zinc oxide complex compound represented bv the general formula (I): 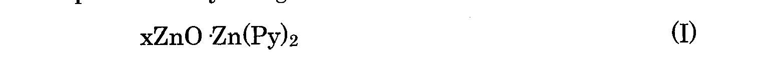 wherein Py represents 2-pyridylthio-N-oxide, and x represents 0 or a positive number, which satisfies a relation: 0 ≤ x ≤ 1, and a titanium oxide hydrate represented by the general formula (II): 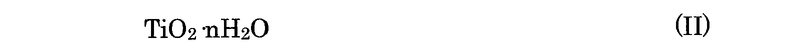 wherein n represents 1 or 2, in a water suspension at a pH of 5 to 10 and a temperature of 10 to 100°C, thereby adsorbing the zinc pyrithione or zinc pyrithione-zinc oxide complex compound to the titanium oxide hydrate;
(6) The process for producing an antibiotic composition according to (5) wherein a weight ratio of the titanium oxide hydrate to the zinc pyrithione or zinc pyrithione-zinc oxide complex compound is from 1 to 50 % by weight;
(7) The process for producing an antibiotic composition according to (5) or (6), wherein x in the general formula (I) satisfies a relation: 0.01 ≤ x ≤ 0.5, and n in the general formula (II) is 1.

The antibiotic composition of the present invention includes a composition which has the anti-microbial activity against Malassezia furfur which cause dandruff on the scalp, and has the effect of preventing adhesion and inhabitation of pollutants in water, such as barnacles, shellfishes and seaweeds which adhere to and inhabit on coating film at ship-bottom and fish-farming nets thereby causing damage to coating film at the ship-bottom and fish-farming nets.
The antibiotic composition of the present invention is an antibiotic composition containing a titanium oxide hydrate in an amount of 1 to 50 % by weight, and preferably 5 to 25% by weight, based on zinc pyrithione or a zinc pyrithione-zinc oxide complex compound of the general formula (I). When the weight ratio of the titanium oxide hydrate to the zinc pyrithione or zinc pyrithione-zinc oxide complex compound is less than the above lower limit, it is impossible to increase the anti-microbial activity against Malassezia furfur and to lower the solubility in seawater by adsorption of the zinc pyrithione or zinc pyrithione-zinc oxide complex compound to the titanium oxide hydrate. Even when the weight ratio exceeds the above upper limit, it is impossible to further increase these effects.

The antibiotic composition of the present invention can be obtained by mixing a powder of the zinc pyrithione or zinc pyrithione-zinc oxide complex compound with a powder of the titanium oxide hydrate, or combining a water-based suspension of the zinc pyrithione or zinc pyrithione-zinc oxide complex compound with a powder or a water-based slurry of the titanium oxide hydrate to give a water-based suspension, followed by stirring at a pH of 5 to 10, preferably 6 to 9, and at a temperature of 10 to 100°C, preferably 20 to 70°C. The stirring time varies depending on an amount to be treated, but is usually from 5 minutes to 4 hours, and preferably from 10 minutes to 2 hours.
As a form of products of the antibiotic composition, a water-based suspension containing 40 to 60% of the antibiotic composition of the present invention is preferred as an anti-dandruff agent for shampoo, while an oil-based suspension such as xylene-based suspension containing 40 to 60% of the antibiotic composition of the present invention is preferred as an underwater antifouling agent for ship-bottom paints.

The zinc pyrithione or zinc pyrithione-zinc oxide complex compound, which constitutes the antibiotic composition of the present invention, is used as it is in the form of a powder, or a water-based suspension or oil-based suspension. In the case of the water-based suspension, a water-based suspension obtained by reacting sodium pyrithione with a zinc salt in a water medium may be used as it is after adjusting the pH within a range from 5 to 10.
The commercially available zinc pyrithione includes, for example, "Clean-Bio ZP" manufactured by Kolon Life Science Inc., "Zinc Omadine Powder" manufactured by Arch Chemicals, Inc., and "TOMICIDE ZPT" manufactured by API Corporation. As the zinc pyrithione-zinc oxide complex compound, those produced by using the synthesis method described in WO 2005/040122 A1 can be used.
x in the above general formula (I) is preferably a positive number which satisfies a relation: 0.01 ≤ x ≤ 0.5, more preferably a relation: 0.02 ≤ x ≤ 0.33.

The titanium oxide hydrate constituting the antibiotic composition of the present invention has the molecular formula: TiO₂·H₂O or TiO₂·2H₂O. When titanium tetrachloride is neutralized at 40°C or lower, for example, 30°C, TiO₂·2H₂O is obtained, while TiO₂·H₂O is obtained when neutralized at a high temperature, for example, 60°C. TiO₂·2H₂O is called ortho-titanic acid, while TiO₂·H₂O is called meta-titanic acid. Particularly, meta-titanic acid TiO₂·H₂O is obtained by hydrolysis under heating of a titanium sulfate solution in the production process of titanium dioxide by a sulfuric acid method, and a commercially available product thereof include, for example, a water-based slurry (containing 30% of titanium oxide by weight) or a powder product "AMT-100" manufactured by TAYCA CORPORATION. Almost all of the particle diameter, as measured by Laser Diffraction/Scattering Particle Size Distribution Analyzer (HORIBA, Ltd. LA-920), of these products is within a range from several tens nm to 2 µm for a water-based slurry product, or within a range from 0.4 to 20 µm for a powder product. The titanium oxide hydrate has a terminal hydroxyl group and a bridge-forming hydroxyl group in a crystal structure, and it is presumed that the hydroxyl groups are combined with the zinc pyrithione or zinc pyrithione-zinc oxide complex compound to form a mild hydrogen bond in the antibiotic composition of the present invention. Furthermore, meta-titanic acid can form a network structure with water thereby lowering the solubility or decreasing leaching rate of the zinc pyrithione or zinc pyrithione-zinc oxide complex compound in seawater. Therefore, in the present invention, meta-titanic acid is more preferable than ortho-titanic acid. When the titanium oxide hydrates are composed of meta-titanic acid and ortho-titanic acid, the content of the meta-titanic acid is preferably 50% by weight, and more preferably 70% by weight or more.

Since the antibiotic composition of the present invention has the anti-microbial activity, which is 2 to 4 times stronger than that of zinc pyrithione, against Malassezia furfur which mainly cause dandruff on the scalp, the amount of zinc pyrithione to be added in an anti-dandruff shampoo or an anti-dandruff hair conditioner can be reduced. Since the antibiotic composition is easily photo-degraded under acidic conditions as compared with zinc pyrithione, the residual amount of zinc pyrithione in sewage treatment facilities in which the solution tends to shift to the acidic side can be decreased. These features are considered to be notable properties since they meet the demands of the present age, such as improvement in safety to consumers and a decrease of an environmental risk. The composition of the present invention is formulated in a shampoo or a hair conditioner in an amount within a range from 0.1 to 1.2 % by weight, and preferably from 0.2 to 1.0% by weight.

As basic materials of the shampoo, anionic, nonionic and amphoteric surfactants with excellent detergency and of high safety are used. As a basic material of the hair conditioner, a cationic surfactant is used. Examples of the surfactants include anionic surfactants such as sodium lauryl sulfate, triethanolamine lauryl sulfate, sodium polyoxyethylene sulfate, and ammonium polyoxyethylene sulfate; nonionic surfactants such as polyoxyethylene sorbitan stearate and polyethylene glycol distearate; amphoteric surfactants such as coconut oil fatty acid amidopropyl betaine; and cationic surfactants such as cetyltrimethylammonium chloride. In addition, purified water, a foaming agent, a thickener, a solubilizer, a flavor, a coloring agent and a preservative may be used and, if desired, a pharmaceutically active component such as dipotassium glycyrrhizinate, and a functional component such as organopolysiloxane may be used.

The solubility of the antibiotic composition of the present invention in seawater accounts for about 60% (in terms of zinc pyrithione) of the solubility of zinc pyrithione. Zinc pyrithione is not necessarily suited for use an antifouling agent of a ship-bottom paint for which long-term antifouling lifetime is required because of its rather quick leaching. Since the lifetime of the antifouling agent to be leached out from a coating film is in inversely proportional to the square of the solubility of the antifouling agent in seawater, the antifouling lifetime of the coating film containing the composition of the present invention is at least 2 times longer than that of zinc pyrithione. Therefore, the antibiotic composition of the present invention can broaden applications of zinc pyrithione as an underwater antifouling agent with longer lifetime. Since the composition containing the zinc pyrithione-zinc oxide complex compound has an excellent stability to copper ions, it can be used in combination with cuprous oxide, and also can broaden the range of applications as the underwater antifouling agent. The ship-bottom antifouling paint can contain the composition in a concentration of 0.1 to 15% by weight, and preferably 1 to 5% by weight, while the fish-farming net antifouling agent can contain the composition in a concentration of 0.1 to 15% by weight, and preferably 1 to 7% by weight.
As a binder of the ship-bottom paint, for example, an acrylic resin, a vinyl resin and chlorinated rubber are used. Particularly, the acrylic resin is preferably one in which an organosilicone group, or an organic acid group via zinc or copper is bonded to a portion of acrylic acid groups so as to impart self-polishing properties, and the resin gradually becomes water soluble by hydrolysis.

Zinc pyrithione as the underwater antifouling agent is effective against algae, but is not necessarily effective against hard organisms such as barnacles. Therefore, it is usually possible to use cuprous oxide, copper rhodanide, zinc oxide, and a triphenylborane compound such as triphenylborane pyridine salt alone or in combination.
In the antibiotic composition of the present invention, it is preferred to use, as the underwater antifouling agent, cuprous oxide, copper rhodanide, zinc oxide and a triphenylborane pyridine salt in combination. It is also possible to use copper pyrithione, zinc ethylenebisdithiocarbamate, zinc dimethyldithiocarbamate and tetramethylthiuram disulfide alone or in combination.
In addition, a solvent such as xylene is used as an indispensable material in a paint formulation, and it is adjusted to a proper pigment volume concentration (PVC) using a color pigment or an extender pigment. If desired, a rosin, a viscosity modifier, an anti-setting agent and an anti-sagging agent may be used so as to control leaching of cuprous oxide and copper rhodanide and to improve coating film properties.

It is possible to use, as the fish-farming net antifouling agent, indispensable material, for example, a binder such as acrylic resin, a solvent such as xylene, and one or more kinds of copper powder, cuprous oxide, glass copper, a triphenylborane compound and zinc oxide, which are effective for preventing adhesion of marine organisms, a dithiocarbamic acid heavy metal salt compound which is particularly useful for preventing adhesion of hydrozoan in combination. It is also possible to use in combination with a leaching adjustment/efficacy enhancing agent such as t-nonyl polysulfide.

### EFFECTS OF THE INVENTION

The antibiotic composition comprising zinc pyrithione, or a zinc pyrithione-zinc oxide complex compound and a titanium oxide hydrate of the present invention has higher anti-dandruff efficacy than that of zinc pyrithione in hair care applications, and can prolong antifouling lifetime as an underwater antifouling agent.

### BEST MODE FOR CARRYING OUT THE INVENTION

The present invention will be described more specifically by way of examples.

### Example 1

9.8 g of a titanium oxide mono-hydrate powder (TiO₂·H₂O) ("AMT-100", manufactured by TAYCA CORPORATION) and 25.6 g of a zinc pyrithione powder ("Clean-Bio ZP", manufactured by Kolon Life Science Inc.) were uniformly mixed and ground by a mixer with a blade to obtain 35.0 g of a composition of zinc pyrithione and titanium oxide hydrate in a weight ratio of 8:1.

### Example 2

1.2 g of a titanium oxide mono-hydrate (TiO₂·H₂O) water-based slurry (containing 30% by weight of titanium oxide, manufactured by TAYCA CORPORATION) and 30 mL of purified water were charged in a 100 mL beaker to give a water-based suspension (pH of about 3). To this water-based suspension, 0.5 g of sodium carbonate was added and, furthermore, 2.6 g of a zinc pyrithione powder (manufactured by Arch Chemicals, Inc.) and 20 mL of purified water were added, followed by stirring under light screening condition at 20°C for 30 minutes (pH of about 8.5). The suspension was filtered through No. 2 filter paper, washed with 30 mL of purified water and then dried under light screening condition to obtain 2.7 g of a white powder. 800 mg of the resultant white powder was added to 500 mL of chloroform in a 1 L three-necked flask, followed by stirring at 60°C for 30 minutes and further filtration with a membrane filter. The filtrate was distilled off to obtain 670 mg of the residue. The amount of the filtration residue was 120 mg.
The resultant distillation residue was subjected to X-ray fluorescence spectrometry. As a result, titanium was not detected. Therefore, it is considered that zinc pyrithione and titanium oxide hydrate do not form a complex compound such as a zinc pyrithione-zinc oxide complex compound in which both components are extracted together from chloroform.

### Example 3

40 g of a zinc pyrithione powder (manufactured by Kolon Life Science Inc.) and 1,500 mL of purified water were charged in a 5 L flask and 16 g of a titanium oxide mono-hydrate (TiO₂·H₂O) water-based slurry (containing 30% by weight of titanium oxide, manufactured by TAYCA CORPORATION) was added, and the pH was adjusted to 8 with an aqueous 1% sodium hydroxide solution, followed by stirring under light screening condition at 20°C for 60 minutes. The operation of filtration through No. 2 filter paper, returning to 500 ml of purified water and filtration was repeated three times. The resultant white solid was dried under light screening condition at 50°C for 5 hours to obtain 42 g of a white powder.

### Example 4

In the same manner as in Example 3, except that the pH was adjusted to 9.5 with an aqueous 1% sodium hydroxide solution and stirring was performed under light screening condition at 20°C for 60 minutes, an operation was performed. After drying, 41 g of a white powder was obtained.
In the same manner as in Example 1, extraction from chloroform was performed and the filtrate was distilled off to obtain 710 mg of the residue. The amount of the filtration residue was 90 mg. The resultant extract from chloroform was subjected to X-ray fluorescence spectrometry. As a result, titanium was not detected. Therefore, it is considered that zinc pyrithione and titanium oxide mono-hydrate do not form a complex compound such as a zinc pyrithione-zinc oxide complex compound even when prepared at higher pH.

### Example 5

In a 500 mL round-bottom flask, 90 mL of an aqueous solution containing 1.0 g (0.024 mol) of sodium hydroxide, 35.8 g (0.096 mol) of an aqueous 40% sodium pyrithione solution and 50 mL of water were charged and 200 mL of an aqueous solution containing 17.3 g (0.06 mol) of zinc sulfate heptahydrate was added dropwise under stirring over 60 minutes while maintaining the temperature at 20°C. Furthermore, the pH was adjusted to 9.5 with concentrated hydrochloric acid and stirring was continued at 20°C for 4 hours. The reaction solution was filtered through No.2 filter paper to obtain a solid. It was confirmed that the filtrate is not colored with ferrous ions by backwashing twice with 100 mL of water. The resultant solid was then washed with 70 mL of water, dried at 50°C for 5 hours and then ground to obtain 15.8 g of a zinc pyrithione-zinc oxide complex compound (x = 0.25) as a white powder (A). As a result of DTA/TG analysis, an exothermic peak temperature was 328.7°C (before correction) or 302.0°C (after correction).
In a 1 L flask, 5 g of a titanium oxide mono-hydrate (TiO₂·H₂O) slurry (containing 30% by weight of titanium oxide, manufactured by TAYCA CORPORATION) was added to 400 mL of water and the pH was adjusted to 8 with 1% sodium hydroxide. 12 g of (A) was added, followed by stirring under light screening condition at 20°C for 60 minutes. The water-based suspension was filtered with No. 2 filter paper and backwashed with 200 mL of water, and then the solid collected by filtration was dried under light screening condition at 50°C for 5 hours and then ground. 13 g of a white powder (B) was obtained from the zinc pyrithione-zinc oxide complex compound (A) and the titanium oxide mono-hydrate.

### Example 6

In the same manner as in Example 3, except that the pH of a water-based suspension containing titanium oxide mono-hydrate water-based slurry and a zinc pyrithione powder was adjusted to 7 with an aqueous 1% sodium hydroxide solution, followed by stirring under light screening condition at 60°C for 15 minutes, an operation was performed. After drying, 40 g of a white powder was obtained.

### Example 7

The solubility of samples shown below in artificial seawater was measured.
Artificial seawater (pH of 7.8) was added to each sample shown below to make total amount of 500 mL, followed by shaking at 20°C for 24 hours. After filtration, determination of zinc was performed by subjecting the filtrate to atomic absorption spectrometry.
Sample 1: 110 mg of white powder of Example 2 (zinc pyrithione: titanium oxide mono-hydrate = 8:1, in weight ratio)
Sample 2: 100 g of zinc pyrithione (manufactured by Arch Chemicals, Inc.)
Artificial seawater was prepared according to the formulation shown in Table 1.

**Table 1 Formulation of artificial seawater (total amount: 1 L, pH 7.8)**

| Components | Content (g/L) |
|---|---|
| NaCl | 24.53 |
| MgCl₂ · 6H₂O | 11.11 |
| Na₂SO₄ | 4.09 |
| CaCl₂ | 1.54 |
| KCl | 0.695 |
| NaHCO₃ | 0.201 |
| KBr | 0.100 |
| H₃BO₃ | 0.027 |
| SrCl₂ · 6H₂O | 0.042 |
| NaF | 0.003 |
| Purified water | Balance |

The measurement results are shown in Table 2.

**Table 2 Solubility in artificial seawater I (mg/L)**

| Samples | Quantitative value of zinc (mg/L) | Value in terms of zinc pyrithione (mg/L) |
|---|---|---|
| Samples 1 | 0.70 | 3.40 |
| Samples 2 | 1.16 | 5.64 |

As shown in Table 2, the solubility of the composition comprising zinc pyrithione and titanium oxide mono-hydrate of the present invention is lowered in artificial seawater as compared with zinc pyrithione (reduction rate of 40%)
From the results, it is presumed that the solubility of zinc pyrithione in artificial seawater was remarkably reduced as a result of adsorption of zinc pyrithione to titanium oxide mono-hydrate. That is, it is considered that zinc pyrithione and titanium oxide mono-hydrate do not form a simple mixture, but is a kind of a composite-like composition.

### Example 8

In the same manner as in Example 6, the solubility of samples shown below in artificial seawater was measured.
Sample 3: 110 g of white powder of Example 3 (zinc pyrithione: titanium oxide mono-hydrate = 8:1, in weight ratio)
Sample 4: 110 mg of white powder of Example 4 (zinc pyrithione: titanium oxide mono-hydrate = 8:1, in weight ratio)
Sample 5: 100 mg of zinc pyrithione + 80 mg of titanium oxide mono-hydrate (TiO₂·H₂O) slurry (containing 30% by weight of titanium oxide)(zinc pyrithione: titanium oxide mono-hydrate (in terms of 100%) = 4:1)
Sample 6: 100 mg of zinc pyrithione (manufactured by Kolon Life Science Inc.)
Sample 7: 125 mg of white powder (A) of Example 5+40 mg of titanium oxide mono-hydrate slurry (containing 30 % by weight of titanium oxide)(A: titanium oxide mono-hydrate (in terms of 100%) = 4:1)
Sample 8: 125 g of white powder (A) of Example 5

**Table 3 Solubility in artificial seawater II (mg/L)**

| Samples | Quantitative value of zinc (mg/L) | Value in terms of zinc pyrithione (mg/L) |
|---|---|---|
| Samples 3 | 0.68 | 3.33 (Reduction rate 36%) |
| Samples 4 | 0.94 | 4.61 (Reduction rate 12%) |
| Samples 5 | 0.63 | 3.09 (Reduction rate 41%) |
| Samples 6 | 1.07 | 5.24 |
| Samples 7 | 3.34 | Reduction rate is 11% based on sample 8 |
| Samples 8 | 3.77 | - |

In Table 3, quantitative values of zinc of samples 7 and 8 mean quantitative values of zinc derived from zinc pyrithione and zinc oxide. Therefore, the value in terms of zinc pyrithione in the right column cannot be calculated and is expressed by the symbol "-".
As shown in Table 3, 1) when zinc pyrithione is treated with titanium oxide mono-hydrate at high pH (9.5), the solubility reducing effect of the composition is weakened, in other words, the adsorptive capacity of titanium oxide mono-hydrate to zinc pyrithione is decreased, and 2) it is presumed that the solubility reducing effect is scarcely exerted due to the zinc oxide moiety in the zinc pyrithione-zinc oxide complex compound. That is, it is considered that titanium oxide mono-hydrate has a stronger affinity to zinc pyrithione than to zinc oxide.

### Example 9

The following water suspension samples are charged in a glass bottle, allowed to stand outside for 10 days, dried and ground, and then yellowness (b* value) was measured by a colorimeter.
Sample 9: 1 g of zinc pyrithione + 20 mL of distilled water
Sample 10: 1 g of zinc pyrithione + 400 mg of titanium oxide mono-hydrate (TiO₂·H₂O) slurry (containing 30% by weight of titanium oxide) + 20 mL of distilled water (pH is adjusted to 5 with sodium bicarbonate)
Sample 11: 1 g of A of Example 5 + 20 mL of distilled water (pH is adjusted to 5 with sodium bicarbonate)
Sample 12: 1 g of A of Example 5 + 400 mg of titanium oxide mono-hydrate slurry (containing 30 % by weight of titanium oxide) + 20 mL of distilled water (pH is adjusted to 5 with sodium bicarbonate)
Sample 13: 1 g of zinc pyrithione + 20 mL of artificial seawater
Sample 14: 1.1 g of composition of Example 1 + 20 mL of artificial seawater
Sample 15: 1 g of A of Example 5 + 20 mL of artificial seawater
Sample 16: 1 g of A of Example 5 + 400 mg of titanium oxide mono-hydrate slurry (containing 30 % by weight of titanium oxide) + 20 mL of artificial seawater
The measurement results are shown in Table 4.

**Table 4 Yellowness of powder**

| Samples | Yellowness (b* value) |
|---|---|
| Samples 9 | 8.80 |
| Samples 10 | 9.87 |
| Samples 11 | 5.68 |
| Samples 12 | 8.92 |
| Samples 13 | 11.07 |
| Samples 14 | 10.86 |
| Samples 15 | 8.12 |
| Samples 16 | 8.56 |

As shown in Table 4, 1) zinc pyrithione and a zinc pyrithione-zinc oxide complex compound are easily photo-degraded at the alkali side as compared with the acidic side, 2) the photo-degradation accelerating effect due to titanium oxide mono-hydrate is exerted at pH 5, but is scarcely exerted in the case of artificial seawater, and 3) a zinc pyrithione-zinc oxide complex compound is not so much photo-degraded as zinc pyrithione.

### Example 10

With respect to the composition of zinc pyrithione and titanium oxide mono-hydrate of Example 2, an anti-microbial test (MIC) against Malassezia furfur by a liquid culture method was performed in comparison with zinc pyrithione.
Sample 17: composition of zinc pyrithione (manufactured by Arch Chemicals, Inc.) and titanium oxide mono-hydrate (Example 2)
Sample 18: zinc pyrithione ( manufactured by Arch Chemicals, Inc.)
Test strain: Malassezia furfur NBRC 0656
Test medium: 1% olive oil is added to Sabouraud glucose medium
Test conditions: shaking culture at 30°C for 4 days (120 rpm)

**Table 5 Anti-microbial test I (MIC) (µg/mL)**

| Samples | Malassezia furfur (Cell count:10⁶cfu/mL) | |
|---|---|---|
| | 2 days | 4 days |
| Sample 17: white powder of Example 2 | 0.78 | 1.56 |
| Sample 18: zinc pyrithione (control) | 6.25 | 6.25 |

As shown in Table 5, the composition of zinc pyrithione and titanium oxide mono-hydrate exhibited an anti-microbial activity 4 times stronger against Malassezia furfur than zinc pyrithione.

### Example 11

With respect to the composition of zinc pyrithione and titanium oxide mono-hydrate (Example 3), an anti-microbial test (MIC) against Malassezia furfur by an agar medium was performed in comparison with zinc pyrithione. Sample 19: composition of zinc pyrithione (manufactured by Kolon Life Science Inc.) and titanium oxide mono-hydrate (Example 3)
Sample 20: zinc pyrithione ( manufactured by Kolon Life Science Inc.)
Test strain: Malassezia furfur NBRC 0656
Test medium: YM agar medium added with olive oil
Test conditions: culture at 28°C for 7 days

**Table 6 Anti-microbial test II (MIC) (µg/mL)**

| Samples | Malassezia furfur (Cell count:10⁵cfu/mL) 7 days |
|---|---|
| Sample 19: white powder of Example 3 | 3.12 |
| Sample 20: zinc pyrithione (control)) | 6.25 |

As shown in Table 6, the composition of zinc pyrithione and titanium oxide mono-hydrate exhibited an anti-microbial activity 2 times stronger against Malassezia furfur than zinc pyrithione.

### Example 12

The following materials were uniformly mixed to obtain a ship-bottom antifouling paint (Symbol W means weight).
Copolymer of methyl methacrylate and isopropylsilyl acrylate in component

| | |
|---|---|
| ratio of 2:3 (50% xylene solution) | 36 W% |
| Cuprous oxide | 35 W% |
| Zinc white | 5 W% |
| Composition of B of Example 5 | 5 W% |
| Titanium white | 1 W% |
| Red oxide of iron | 1 W% |
| Fatty acid amide wax (20%) | 2 W% |
| Xylene | 15 W% |
| Total | 100 W% |

### Example 13

The following materials were uniformly mixed to obtain a fish-farming net antifouling agent (Symbol W means weight).
Butyl acrylate/methyl methacrylate copolymer (50 W% xylene solution)

| | |
|---|---|
| | 20 W% |
| Triphenylboran pyridine salt | 6 W% |
| Composition of Example 6 | 2 W% |
| Zinc white | 9 W% |
| Polyether silicone oil | 2 W% |
| t-Nonyl polysulfide | 3 W% |
| Xylene | 58 W% |
| Total | 100 W% |

### Example 14

The following materials were uniformly mixed to obtain an anti-dandruff shampoo (Symbol W means weight).
Sodium polyoxyethylene (EO = 2 mol) lauryl ether sulfate

| | |
|---|---|
| | 16.0 W% |
| Composition of Example 3 | 0.5 W% |
| Propylene glycol | 0.3 W% |
| Citric acid | trace amount |
| Purified water | balance |
| Total | 100.0 W% |

### INDUSTRIAL APPLICABILITY

The antibiotic composition comprising zinc pyrithione or a zinc pyrithione-zinc oxide complex compound represented by the general formula (I) and a titanium oxide hydrate represented by the general formula (II) enhance the anti-microbial activity against Malassezia furfur which cause dandruff on the scalp, and to improve bio-degradability under the acidic condition and to lower the solubility in seawater, and therefore can be used as an excellent anti-dandruff agent and an excellent underwater antifouling agent as compared with zinc pyrithione.

## Claims

1. An antibiotic composition comprising zinc pyrithione or a zinc pyrithione-zinc oxide complex compound represented by the general formula (I): 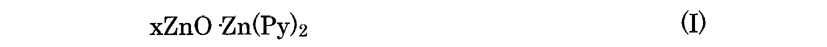 wherein Py represents 2-pyridylthio-N-oxide, and x represents 0 or a positive number, which satisfies a relation: 0 ≤ x ≤ 1, and a titanium oxide hydrate represented by the general formula (II): 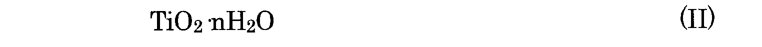 wherein n represents 1 or 2.

2. The antibiotic composition according to claim 1, which is an anti-dandruff agent.

3. The antibiotic composition according to claim 1, which is an underwater antifouling agent.

4. The antibiotic composition according to claim 3, which further contains a binder, an inorganic copper compound, and/or an inorganic zinc compound, and/or copper pyrithione, and/or a triphenylborane compound.

5. A process for producing an antibiotic composition, which comprises stirring zinc pyrithione or a zinc pyrithione-zinc oxide complex compound represented by the general formula (I): 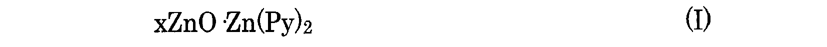 wherein Py represents 2-pyridylthio- N-oxide, and x represents 0 or a positive number, which satisfies a relation: 0 ≤ x ≤ 1, and a titanium oxide hydrate represented by the general formula (II): 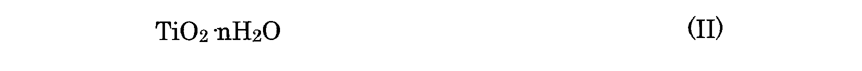 wherein n represents 1 or 2, in a water suspension at a pH of 5 to 10 and a temperature of 10 to 100°C, thereby adsorbing the zinc pyrithione or zinc pyrithione-zinc oxide complex compound to the titanium oxide hydrate.

6. The process for producing an antibiotic composition according to claim 5, wherein a weight ratio of the titanium oxide hydrate to the zinc pyrithione or zinc pyrithione-zinc oxide complex compound is from 1 to 50 % by weight.

7. The process for producing an antibiotic composition according to claim 5 or 6, wherein x in the general formula (I) satisfies a relation: 0.01 ≤ x ≤ 0.5, and n in the general formula (II) is 1.
